# EUROPEAN PATENT APPLICATION

(11) **EP 1 066 845 A1**
(43) Date of publication of application: **10.01.2001**
(21) Application number: 99900172.0
(22) Date of filing: 12.01.1999
(51) Int. Cl.: A61M 5/14, A61M 5/152, A61M 5/162

(54) **CONTINUOUS TRANSFUSION INJECTION UNIT ASSEMBLY**

(71) Applicant: TSUKADA MEDICAL RESEARCH CO., LTD., Tokyo 169-0074 (JP)
(72) Inventor: TSUKADA, Osamu, Ueda-shi, Nagano 386-0002 (JP)
(74) Representative: Spall, Christopher John
(86) International application number: JP9900067
(87) International publication number: WO0041750

(57) **Abstract**

A continuous injection unit assembly for a transfusive liquid comprises a bag unit (1) for a transfusive liquid made of a flexible material, adapted to contain a transfusive liquid therein, and having an elastic extractive portion (11); a continuous injector unit (2) for a transfusive liquid having an inlet portion (21), an outlet portion (22), and an elastic balloon (23) which interconnects the inlet and outlet portions (21, 22), a transfusive liquid injected in the balloon (23) through the inlet portion (21) in the continuous injector unit (2) being caused to flow out of the outlet portion (22) continuously over a long period of time by means of an elastic contractive force exerted in the elastic balloon (23); and a connector unit (3) for detachably interconnecting the elastic extractive portion (11) of the bag unit (1) and the inlet portion (21) of the continuous injector unit (2). The transfusive liquid is rapidly and conveniently transferred from the bag unit (1) for a transfusive liquid through the connector unit (3) into the continuous injector unit (2) for a transfusive liquid.

## Description

### TECHNICAL FIELD

This invention relates to a continuous injection unit assembly for a transfusive liquid which transfers a transfusive liquid from a transfusive liquid bag unit to a continuous injector unit for a transfusive liquid which has a function of discharging the transfusive liquid for a long period of time.

### BACKGROUND ART

Heretofore, there is in a part of a transfusive liquid set a transfusive liquid bag unit made of a flexible transparent material (for example, polyethylene, polypropylene, or the like). The transfusive liquid bag unit is charged with a transfusive liquid (for example, physiological saline solution, grape sugar solution, antibiotic substance solution, calmative solution, analgesic solution, heparin solution, nitroglycerin solution, or the like). The transfusive liquid bag unit has an elastic outlet portion for a transfusive liquid at one end thereof and an aperture for hanging at the other end thereof.

In the case where the transfusive liquid set is used in an intravenous drip treatment, an intravenous drip unit is coupled to the outlet portion of the transfusive liquid bag unit and the transfusive liquid is continuously injected into a human body or the like. Since the transfusive liquid set restricts the movement of a patient, it has caused inconvenience to a patient and a nursing person.

On the other hand, there has been a continuous injector for a liquid medicine (Japanese Patent No. 1384289) developed by the present applicant. This injector includes an inlet portion for a liquid medicine, an outlet portion for a liquid medicine, and a balloon which connects the inlet portion to the outlet portion. The liquid medicine injected in the balloon flows out through the outlet portion for a long period of time.

However, this continuous injector for a liquid medicine requires an additional injector for injecting a liquid medicine into the balloon of the former injector. This operation is troublesome and maintenance of the pair of injectors is inconvenient. Thus, the present applicant has proposed "a continuous injector for a liquid medicine with a bellows type container" (Japanese Utility Model Publication No. 6-7722 (1994)). Although injection of a liquid medicine has become convenient according to the injector, it is difficult to precisely set a volume of a liquid medicine by means of the bellows type container and there has been no existing connector which can readily interconnect the balloon and the injector for a liquid medicine.

### DISCLOSURE OF INVENTION

An object of the present invention is to provide an assembly of units which can easily inject a given amount of a medical liquid into the continuous injector for a liquid medicine disclosed in the Japanese Patent mentioned above.

A continuous injection unit assembly for a transfusive liquid in accordance with the present invention comprises a bag unit for a transfusive liquid made of a flexible material, adapted to contain a transfusive liquid therein, and having an elastic extractive portion; a continuous injector unit for a transfusive liquid having an inlet portion, an outlet portion, and an elastic balloon which interconnects the inlet and outlet portions, a transfusive liquid injected in the balloon through the inlet portion in the continuous injector unit being caused to flow out of the outlet portion continuously over a long period of time by means of an elastic contractive force exerted in the elastic balloon; and a connector unit for detachably interconnecting the elastic extractive portion of the bag unit and the inlet portion of the continuous injector unit. The transfusive liquid is rapidly and conveniently transferred from the transfusive liquid bag unit through the connector unit into the continuous injection unit for a transfusive liquid.

The connector unit may be provided on an end thereof with a piercing needle portion and on the other end with a female lure portion having a check valve, may be provided on an end thereof with a piercing needle portion and on the other end with a female lure portion having a check valve and a male-screwed flange, may be provided on an end thereof with a piercing needle portion and on the other end with a tapered male lure portion, may be provided on an end thereof with a piercing needle portion and on the other end with a female lure portion having a male-screwed flange, or may be provided on an end thereof with a piercing needle portion and on the other end with a male lure portion having a female-screwed flange.

The piercing needle portion on the end of the connector unit penetrates the elastic extractive portion of the bag unit for a transfusive liquid to communicate with an interior of the bag unit while the female or male lure portion on the other end of the connector unit couples to the inlet portion of the continuous injection unit for a transfusive liquid.

### BRIEF DESCRIPTION OF DRAWINGS

FIG. 1 is a longitudinal sectional view of a bag unit for a transfusive liquid constituting a part of an assembly in accordance with the present invention;
FIG. 2 is a plan view of a connector unit constituting a part of the assembly in accordance with the present invention, illustrating different kinds of alterations;
FIG. 3 is a plan view of a continuous injector unit constituting a part of the assembly in accordance with the present invention, illustrating the unit in which a transfusive liquid is injected;
FIG. 4 is an explanatory view of typical examples of instruments to be attached to a human body and to be connected to an outlet portion of the continuous injection unit for a transfusive liquid; and
FIG. 5 is a plan view of an example of use of the assembly in accordance with the present invention;

### BEST MODE FOR CARRYING OUT THE INVENTION

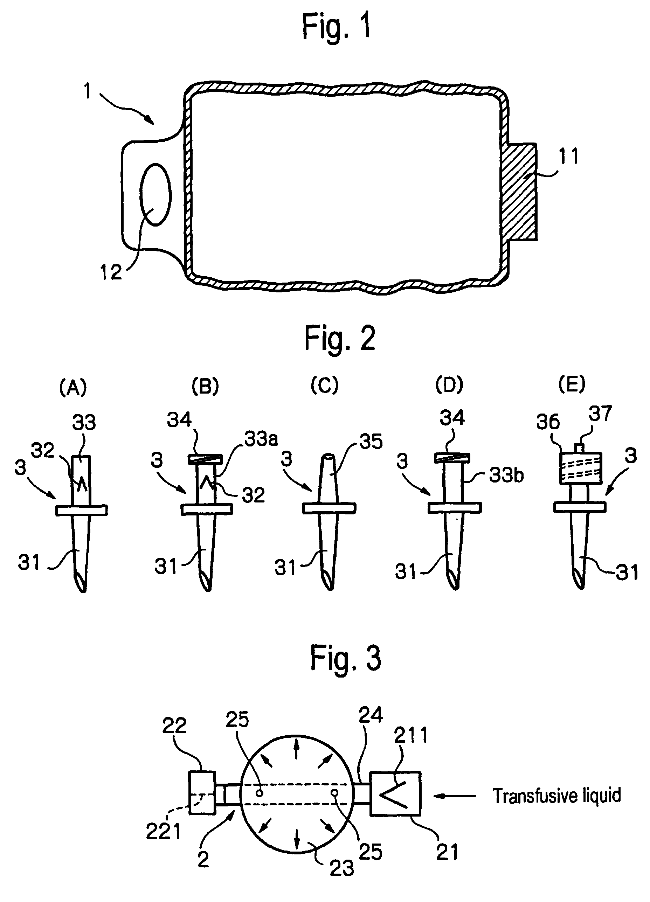

An embodiment of a continuous injection unit assembly for a transfusive liquid in accordance with the present invention will be explained below by referring to FIGS. 1 to 3.

A continuous injection unit assembly in accordance with the present invention, as shown in FIGS. 1 to 3, comprises a bag unit 1 for a transfusive liquid, a continuous injector unit 2 for a transfusive liquid, and a connector unit 3.

The bag unit 1 for a transfusive liquid is made of a flexible transparent material (for example, polyethylene, polypropylene, or the like). The transfusive liquid bag unit 1 is charged with a transfusive liquid (for example, physiological saline solution, grape sugar solution, antibiotic substance solution, calmative solution, analgesic solution, heparin solution, nitroglycerin solution, or the like). The transfusive liquid bag unit 1 has an elastic extractive outlet portion 11 for a transfusive liquid at one end thereof and an aperture 12 for hanging at the other end thereof.

The continuous injector unit 2 for a transfusive liquid, as shown in FIG. 3, is provided on opposite ends of a cylindrical body 21 with an inlet portion 21 for a transfusive liquid and an outlet portion 22 for a transfusive liquid. A balloon 23 is attached to an outer periphery of the cylindrical body 24. An interior of the balloon 23 is communicated with an interior of the cylindrical body 24 through a communication hole 25.

The cylindrical body 24 may be omitted and the inlet portion 21 and outlet portion 22 may be directly interconnected.

The inlet portion 21 is provided with a check valve 211 which prevents the injected transfusive liquid from flowing in a reverse direction. The balloon 23 is made of an elastic material and can accommodate a given amount of a transfusive liquid. The outlet portion 22 is provided with a control path 221 which controls an outflow period of time for a transfusive liquid.

Thus, the continuous injection unit 2 for a transfusive liquid has a function of flowing the transfusive liquid injected into the balloon 23 through the inlet portion 21 out of the outlet portion 22 over a long period of time by an elastic contractive force of the balloon 23 itself.

The connector unit 3, as shown In FIG. 2, may be provided on an end thereof with a piercing needle portion 31 and on the other end with a female lure portion 33 having a check valve 32 (A), may be provided on an end thereof with a piercing needle portion 31 and on the other end with a female lure portion 32a having a check valve 32 and a male-screwed flange 34 (B), may be provided on an end thereof with a piercing needle portion 31 and on the other end with a tapered male lure portion 35 (C), may be provided on an end thereof with a piercing needle portion 31 and on the other end with a female lure portion 33b having a male-screwed flange 34 (D), or may be provided on an end thereof with a piercing needle portion 31 and on the other end with a male lure portion 37 having a female-screwed flange 36 (E).

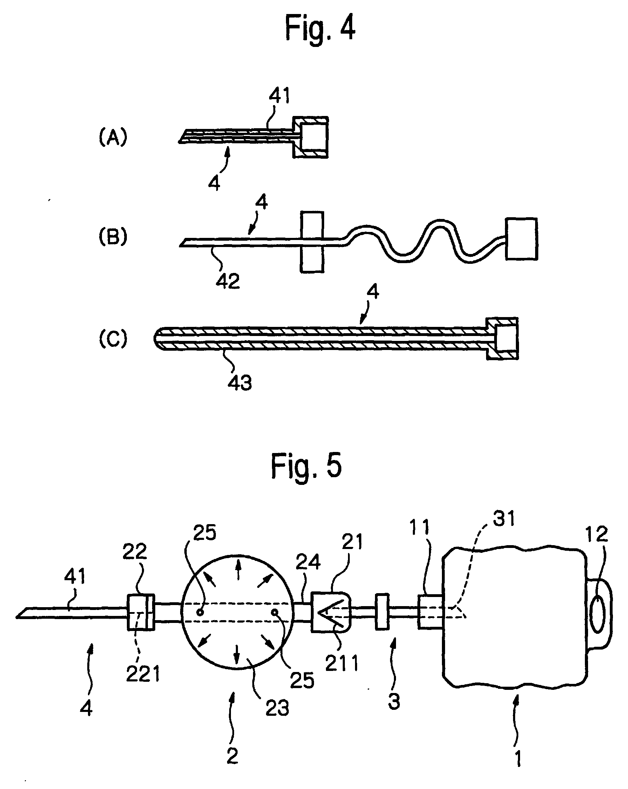

FIG. 4 shows a typical example of an instrument 4 to be attached to a human body. The instrument 4 is connected to the outlet portion 22 of the continuous injector unit 2 for a transfusive liquid. (A) illustrates a usual injection needle 41, (B) illustrates an injection needle 42 having a flexible tube, and (C) illustrates a usual catheter 43.

An example of use of the assembly in accordance with the present invention is shown in FIG. 5. The piercing needle portion 31 on the end of the connector unit 3 penetrates the elastic extractive portion 11 of the transfusive liquid bag unit 1 to communicate with an interior of the bag unit 1 while the female or male lure portion 33 or 37 on the other end of the connector unit 3 couples to the inlet portion 21 of the continuous injector unit 2 for a transfusive liquid. The instrument 4 to be attached to a human body (for example, the injection needle 41 in FIG. 5) is coupled to the outlet portion 22 of the unit 2.

A given amount (for example, 50 cc, 100 cc, 200 cc, or 500 cc) is predeterminately contained and sealed in the transfusive liquid bag unit 1 in accordance with a kind of a transfusive liquid. A medical doctor or person selects the unit 1 in accordance with a treatment condition of a patient and can easily and rapidly transfer the transfusive liquid from the unit 1 into the unit 2 by manually pressing the unit 1.

### INDUSTRIAL APPLICABILITY

According to the present invention, it is possible not only to widen a range of use for a continuous injector unit for a transfusive liquid, to simplify and enhance conventional medical treatment but also to minimize a residual amount of transfusive liquid in the container. In particular, the assembly of the present invention is effective for first-aid treatment of emergency patients.

## Claims

1. A continuous Injection unit assembly for a transfusive liquid, comprising:
a bag unit (1) for a transfusive liquid made of a flexible material, adapted to contain a transfusive liquid therein, and having an elastic extractive portion (11);
a continuous injector unit (2) for a transfusive liquid having an inlet portion (21), an outlet portion (22), and an elastic balloon (23) which interconnects said inlet and outlet portions (21, 22), a transfusive liquid injected in said balloon (23) through said inlet portion (21) in said continuous injector unit (2) being flowed out of said outlet portion (22) continuously over a long period of time by means of an elastic contractive force exerted in said elastic balloon (23); and
a connector unit (3) for detachably interconnecting said elastic extractive portion (11) of said bag unit (1) and said inlet portion (21) of said continuous injector unit (2).

2. A continuous injection unit assembly for a transfusive liquid according to Claim 1, wherein said connector unit (3) is provided on an end thereof with a piercing needle portion (31) and on the other end with a female lure portion (33) having a check valve (32).

3. A continuous injection unit assembly for a transfusive liquid according to Claim 1, wherein said connector unit (3) is provided on an end thereof with a piercing needle portion (31) and on the other end with a female lure portion (33a) having a check valve (32) and a male-screwed flange (34).

4. A continuous injection unit assembly for a transfusive liquid according to Claim 1, wherein said connector unit (3) is provided on an end thereof with a piercing needle portion (31) and on the other end with a tapered male lure portion (35).

5. A continuous injection unit assembly for a transfusive liquid according to Claim 1, wherein said connector unit (3) is provided on an end thereof with a piercing needle portion (31) and on the other end with a female lure portion (33b) having a male-screwed flange (34).

6. A continuous injection unit assembly for a transfusive liquid according to Claim 1, wherein said connector unit (3) is provided on an end thereof with a piercing needle portion (31) and on the other end with a male lure portion (37) having a female-screwed flange (36).
